# EUROPEAN PATENT APPLICATION

(11) **EP 4 074 332 A1**
(43) Date of publication of application: **19.10.2022**
(21) Application number: 20897752.0
(22) Date of filing: 09.12.2020
(51) Int. Cl.: A61K 39/00

(54) **METHOD FOR PREPARING PEPTIDE EMULSION FORMULATION**

(30) Priority: 10.12.2019 JP 2019223030
(71) Applicant: Sumitomo Pharma Co., Ltd., Osaka 541-0045 (JP)
(72) Inventor: FUJII, Tetsuya, Osaka-shi, Osaka 554-0022 (JP); MORITA, Akihiro, Suzuka-shi, Mie 513-0818 (JP); NAKATANI, Tomomi, Suita-shi, Osaka 564-0053 (JP); TSUZUKU, Takuma, Tokyo 104-8356 (JP)
(74) Representative: Vossius & Partner Patentanwälte Rechtsanwälte mbB
(86) International application number: PCT/JP2020/045878
(87) International publication number: WO 2021/117771

(57) **Abstract**

Provided is a method for preparing a peptide emulsion formulation capable of preparing a desired peptide emulsion formulation. A method for preparing a peptide emulsion formulation includes a step of mixing an aqueous solution containing a compound consisting of an amino acid sequence represented by Formula (1), a pharmaceutically acceptable salt thereof, and a peptide consisting of an amino acid sequence represented by WAPVLDFAPPGASAYGSL (SEQ ID NO: 1) or a pharmaceutically acceptable salt thereof with an oily formulation and applying vibration mixing; and a membrane emulsification step of passing a premixed solution after the vibration mixing through a membrane filter to emulsify the premixed solution.

SEQUENCE LISTING

<110>  Sumitomo Dainippon Pharma Co., Ltd.

<120>  Method for preparing peptide emulsion formulation

<130>  675936

<150>  JP 2019-223030
<151>  2019-12-10

<160>  7

<170>  PatentIn version 3.5

<210>  1
<211>  18
<212>  PRT
<213>  Artificial Sequence

<220>
<223>  Peptide

<400>  1

Trp Ala Pro Val Leu Asp Phe Ala Pro Pro Gly Ala Ser Ala Tyr Gly
1               5                   10                  15

Ser Leu

<210>  2
<211>  19
<212>  PRT
<213>  Artificial Sequence

<220>
<223>  Peptide

<400>  2

Cys Trp Ala Pro Val Leu Asp Phe Ala Pro Pro Gly Ala Ser Ala Tyr
1               5                   10                  15

Gly Ser Leu

<210>  3
<211>  19
<212>  PRT
<213>  Artificial Sequence

<220>
<223>  Peptide

<400>  3

Trp Ala Pro Val Leu Asp Phe Ala Pro Pro Gly Ala Ser Ala Tyr Gly
1               5                   10                  15

Ser Leu Cys

<210> 4
<211> 9
<212> PRT
<213> Artificial Sequence

<220>
<223> Peptide

<400> 4

Arg Met Phe Pro Asn Ala Pro Tyr Leu
1               5

<210> 5
<211> 10
<212> PRT
<213> Artificial Sequence

<220>
<223> Peptide

<400> 5

Cys Arg Met Phe Pro Asn Ala Pro Tyr Leu
1               5                   10

<210> 6
<211> 9
<212> PRT
<213> Artificial Sequence

<220>
<223> Peptide

<400> 6

Cys Met Thr Trp Asn Gln Met Asn Leu
1               5

<210> 7
<211> 9
<212> PRT
<213> Artificial Sequence

<220>
<223> Peptide

<400> 7

Cys Tyr Thr Trp Asn Gln Met Asn Leu
1               5

## Description

### TECHNICAL FIELD

The present invention relates to a method for preparing a peptide emulsion formulation.

### BACKGROUND ART

"Emulsification" is a process of making one of two liquids that are not mixed with each other into fine particles and dispersing them in the other, and a formulation in which one of the two liquids that are not mixed with each other by this emulsification is dispersed in the form of fine particles in another liquid is called an emulsion formulation. This emulsion formulation is widely used in the food industry, the agrochemical industry, the cosmetic industry, and the like, and is often used as an external preparation, an oral preparation, and an injection in the pharmaceutical industry. In particular, the emulsion formulation in which a continuous phase is oil and a discontinuous phase is water droplets is referred to as a W/O type emulsion formulation, and the emulsion formulation in which the continuous phase is water and the discontinuous phase is oil droplets is referred to as an O/W type emulsion formulation.

In the pharmaceutical industry, it is very important from the viewpoint of drug efficacy, safety, and quality that the W/O type emulsion formulation and the O/W type emulsion formulation are selectively used according to the purpose, and are maintained in the selected form in many cases. In particular, when a hydrophilic drug such as a hydrophilic peptide or protein is dispersed in an oil, a W/O type emulsion formulation is often used.

Examples of a method for preparing the W/O type emulsion formulation include a method for dispersing a liquid for forming a discontinuous phase by press-fitting the liquid into a liquid of a continuous phase with a piston or the like, a method for dispersing a liquid for forming a discontinuous phase by press-fitting the liquid into a liquid of a continuous phase with a piston or the like via a porous body, and a method for alternately reciprocating a mixed solution of a plurality of drug solutions between two syringes.

For example, Patent Literature 1 discloses a device for preparing an emulsion, the device including a filter part, in which the filter part includes first and second mesh parts and fibers, and the fibers are filled in a space between the first mesh part and the second mesh part to form a fiber assembly. In addition, Patent Literature 2 discloses a device for preparing an emulsion in which a porous body is provided in a conduit part formed to be communicable with a pump-type container as a device for preparing an emulsion capable of easily preparing an emulsion even in a place where an emulsifying device is not provided, for example, a general home. In particular, it discloses a device for preparing an emulsion, which is a device capable of preparing an emulsion in a palm, in which (a) a disc-shaped porous body is sandwiched between the facing connectors, (b) syringes for injection are connectable on both sides, (c) when the porous body is hydrophilic, an O/W type emulsion can be produced, and (d) alternatively, when the porous body is hydrophobic, a W/O type emulsion can be prepared.

Patent Literature 3 discloses a method for producing an emulsified oil-and-fat composition in which an emulsified oil-and-fat composition having an average particle diameter of 1 to 20 times a pore diameter of a porous membrane having a uniform pore diameter is prepared in advance, and the emulsified oil-and-fat composition is passed through the porous membrane having the uniform pore diameter to be re-emulsified. In addition, Patent Literature 4 discloses a method for producing an emulsion in which a liquid to be a dispersed phase is press-fitted into a liquid to be a continuous phase through a microporous membrane body having a uniform pore diameter as a simple method for producing emulsion particles in which the diameter can be uniformly and optionally controlled.

Further, Patent Literature 5 discloses a method for preparing a physiologically active peptide emulsion formulation, in which a mixed solution containing at least a physiologically active aqueous peptide solution and an oil adjuvant and having a content ratio of the oil adjuvant to the physiologically active aqueous peptide solution of 0.5 to 5 volume times is prepared, and the mixed solution is moved between two syringes connected by a connector.

### CITATION LIST

### PATENT LITERATURES

Patent Literature 1: WO 2013/133209 A
Patent Literature 2: JP 2005-186026 A
Patent Literature 3: JP H 06-039259 A
Patent Literature 4: JP H 02-095433 A
Patent Literature 5: WO 2007/083763 A

### SUMMARY OF THE INVENTION

### TECHNICAL PROBLEMS

As described above, several methods for emulsifying a drug solution have been proposed. However, when a drug solution containing a peptide is used, depending on the type of the peptide contained in the drug solution, emulsification cannot be performed, and even if the emulsification can be performed, there is a problem that separation is easy after preparation into a peptide emulsion formulation. When a known method is employed to obtain an emulsion, the emulsion cannot be prepared, and the prepared emulsion is unstable or the process is very complicated. Therefore, it is required to more easily obtain a stable emulsion in a short time.

Furthermore, in Patent Literature 3, a stirring type emulsifier, for example, a chemical stirrer or a homomixer is used for preliminary emulsification. However, from the viewpoint of hygiene that impurities derived from the filter part or the porous body should be reduced as much as possible and from the viewpoint of improving workability, in recent years, it has been required to perform stirring without immersing a stirring bar in a drug solution, and it has been required to perform simple preparation using a disposable preparation tool.

The present invention has been made in view of these circumstances, and an object thereof is to provide a method for preparing a desired peptide emulsion formulation, preferably a method capable of easily preparing a desired peptide emulsion formulation in a short time.

### SOLUTION TO PROBLEMS

As a result of intensive studies on a method for preparing a peptide emulsion formulation containing a desired peptide, the present inventors have found that it is only required to include a step of mixing an aqueous solution containing two types of peptides and an oily formulation, and then applying vibration mixing, and a membrane emulsification step of passing a premixed solution after the vibration mixing through a membrane filter to emulsify the premixed solution.

That is, the present invention relates to the following.

[1] A method for preparing a peptide emulsion formulation including:
   a step of mixing an aqueous solution containing a compound consisting of an amino acid sequence represented by Formula (1): 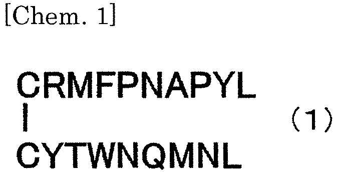
   (wherein a bond between C and C represents a disulfide bond.) or a pharmaceutically acceptable salt thereof, and a peptide consisting of an amino acid sequence represented by WAPVLDFAPPGASAYGSL (SEQ ID NO: 1) or a pharmaceutically acceptable salt thereof with an oily formulation and applying vibration mixing; and
   a membrane emulsification step of passing a premixed solution after the vibration mixing through a membrane filter to emulsify the premixed solution.
[2] The method for preparing a peptide emulsion formulation according to [1], wherein in the membrane emulsification step, the premixed solution after the vibration mixing is caused to pass through the membrane filter once or twice or more to emulsify the premixed solution.
[3] The method for preparing a peptide emulsion formulation according to [1], wherein in the membrane emulsification step, the premixed solution after the vibration mixing is caused to pass through the membrane filter once to emulsify the premixed solution.
[4] The method for preparing a peptide emulsion formulation according to any one of [1] to [3], further including: a step of reciprocating an emulsion obtained in the membrane emulsification step once or several times or more between two containers connected without passing through a membrane filter.
[5] The method for preparing a peptide emulsion formulation according to [4], wherein the number of reciprocations is two or more.
[6] The method for preparing a peptide emulsion formulation according to [4] or [5], wherein the number of reciprocations is 30 or less.
[7] The method for preparing a peptide emulsion formulation according to any one of [4] to [6], wherein the number of reciprocations is 20 or less.
[8] The method for preparing a peptide emulsion formulation according to any one of [4] to [7], wherein the number of reciprocations is 10 or less.
[9] The method for preparing a peptide emulsion formulation according to [4], wherein the number of reciprocations is one.
[10] The method for preparing a peptide emulsion formulation according to any one of [1] to [9], wherein the vibration mixing is shaking by hand.
[11] The method for preparing a peptide emulsion formulation according to any one of [1] to [10], wherein the membrane filter is formed of a component containing one or more selected from the group consisting of polyamide, polyester, fluororesin, polyethersulfone, polypropylene, cellulose acetate, cellulose mixed ester, and glass fiber.
[12] The method for preparing a peptide emulsion formulation according to any one of [1] to [11], wherein the membrane filter is formed of a component containing one or more selected from the group consisting of polyamide, polyester, fluororesin, polypropylene, cellulose acetate, and glass fiber.
[13] The method for preparing a peptide emulsion formulation according to any one of [1] to [12], wherein the membrane filter is formed of one layer or a plurality of layers in which layers of the same components or different components overlap each other.
[14] The method for preparing a peptide emulsion formulation according to any one of [11] to [13], wherein the fluororesin is one or more selected from the group consisting of polytetrafluoroethylene and polyvinylidene fluoride.
[15] The method for preparing a peptide emulsion formulation according to any one of [11] to [14], wherein the fluororesin is polytetrafluoroethylene.
[16] The method for preparing a peptide emulsion formulation according to any one of [1] to [15], wherein the oily formulation is incomplete Freund's adjuvant.
[17] The method for preparing a peptide emulsion formulation according to any one of [1] to [16], wherein the oily formulation is Montanide.
[18] The method for preparing a peptide emulsion formulation according to any one of [1] to [17], wherein the peptide emulsion formulation is a W/O type peptide emulsion formulation.
[19] The method for preparing a peptide emulsion formulation according to any one of [1] to [18], wherein the peptide emulsion formulation contains an excipient.
[20] The method for preparing a peptide emulsion formulation according to [19], wherein the excipient contains one or more selected from the group consisting of purified sucrose, glycine, lactose, glucose, maltose, sodium chloride, sucrose, mannitol, trehalose, and trehalose hydrate.
[21] The method for preparing a peptide emulsion formulation according to any one of [1] to [20], wherein the peptide emulsion formulation contains a pH adjusting agent.
[22] The method for preparing a peptide emulsion formulation according to [21], wherein the pH adjusting agent contains one or more selected from the group consisting of citric acid, lactic acid, tartaric acid, hydrochloric acid, sulfuric acid, nitric acid, succinic acid, sodium hydroxide, potassium hydroxide, tromethamol, histidine, L-arginine, and meglumine.
[23] The method for preparing a peptide emulsion formulation according to [21] or [22], wherein the pH adjusting agent contains one or more selected from the group consisting of tartaric acid, succinic acid, hydrochloric acid, sodium hydroxide, trometamol, L-arginine, and meglumine.
[24] The method for preparing a peptide emulsion formulation according to any one of [1] to [23], wherein the peptide emulsion formulation contains an antioxidant.
[25] The method for preparing a peptide emulsion formulation according to [24], wherein the antioxidant contains one or more selected from the group consisting of methionine, ascorbic acid, sodium edetate, and sodium pyrosulfite.
[26] The method for preparing a peptide emulsion formulation according to [24] or [25], wherein the antioxidant is methionine.
[27] The method for preparing a peptide emulsion formulation according to any one of [1] to [26], wherein the peptide emulsion formulation contains a solubilizer.
[28] The method for preparing a peptide emulsion formulation according to [27], wherein the solubilizer contains one or more selected from the group consisting of natural cyclodextrins such as α-cyclodextrin, β-cyclodextrin, γ-cyclodextrin, hydroxypropyl β-cyclodextrin, and sulfobutyl ether β-cyclodextrin, cyclodextrin derivatives such as hydroxyethyl-β-cyclodextrin (HE-β-CD), hydroxypropyl-β-cyclodextrin (HP-β-CD), methyl-β-cyclodextrin (M-β-CD), and sulfobutyl ether-β-cyclodextrin (SBE-β-CD), amines such as ethylenediamine, triethanolamine, and diethanolamine, acids such as aspartic acid, glycine, phosphoric acid, tartaric acid, acetic acid, citric acid, succinic acid, sodium L-arginine deoxycholic acid, and ursodesoxycholic acid, urea, ethyl urea, meglumine, ethanol, propylene glycol, polyethylene glycol, sodium salicylate, and nicotinic acid amide.
[29] The method for preparing a peptide emulsion formulation according to [27] or [28], wherein the solubilizer contains one or more selected from the group consisting of β-cyclodextrin, hydroxypropyl-β-cyclodextrin (HP-β-CD), tartaric acid, and succinic acid.
[30] A peptide emulsion formulation that is stabilized in a long period of time, containing:
   an aqueous solution containing a compound consisting of an amino acid sequence represented by Formula (1): 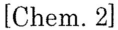 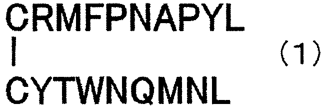
   (wherein a bond between C and C represents a disulfide bond.) or a pharmaceutically acceptable salt thereof, and a peptide consisting of an amino acid sequence represented by WAPVLDFAPPGASAYGSL (SEQ ID NO: 1) or a pharmaceutically acceptable salt thereof; and an oily formulation.
[31] A kit for preparing a peptide emulsion formulation that is stabilized in a long period of time, containing:
   an aqueous solution containing a compound consisting of an amino acid sequence represented by Formula (1): 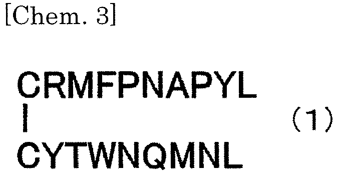
   (wherein a bond between C and C represents a disulfide bond.) or a pharmaceutically acceptable salt thereof, and a peptide consisting of an amino acid sequence represented by WAPVLDFAPPGASAYGSL (SEQ ID NO: 1) or a pharmaceutically acceptable salt thereof; an oily formulation; and membrane filter.
[32] A device for preparing an emulsion at least including:
   a membrane filter for preparing a peptide emulsion formulation that is stabilized in a long period of time,
   wherein the peptide emulsion formulation contains
   an aqueous solution containing a compound consisting of an amino acid sequence represented by Formula (1): 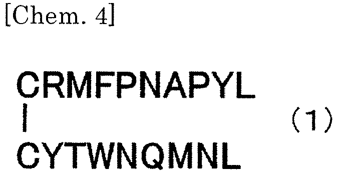
   (wherein a bond between C and C represents a disulfide bond.) or a pharmaceutically acceptable salt thereof, and a peptide consisting of an amino acid sequence represented by WAPVLDFAPPGASAYGSL (SEQ ID NO: 1) or a pharmaceutically acceptable salt thereof; and an oily formulation.

### ADVANTAGEOUS EFFECTS OF INVENTION

According to the present invention, it is possible to easily prepare a desired peptide emulsion formulation which is not easily prepared by a known method.

### BRIEF DESCRIPTION OF THE DRAWINGS

Fig. 1 is a diagram illustrating an example of a profile of transmittance in examples.
Fig. 2 is a diagram illustrating a profile of transmittance in examples for each material of a filter and the number of times of pumping.
Fig. 3 is a diagram illustrating a profile of transmittance in examples for each material of a filter and the number of times of pumping.
Fig. 4 is a diagram illustrating a profile of transmittance for each number of times of pumping when a filter made of polyamide is used in examples.

### DESCRIPTION OF EMBODIMENTS

The present inventors have intensively studied to realize a method for preparing a desired peptide emulsion formulation, preferably a method for preparing a peptide emulsion formulation capable of easily preparing a desired peptide emulsion formulation in a short time. Among the peptides, from the viewpoint that it is difficult to prepare a peptide emulsion formulation when the peptide is mixed with an oily formulation, and the peptide emulsion formulation is hardly stabilized and is easily separated in a short time after emulsification, the present inventors particularly have conducted diligent research on a combination with a compound consisting of an amino acid sequence represented by Formula (1): 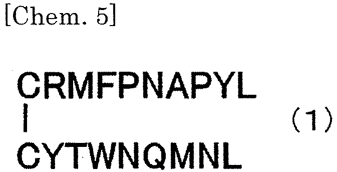
(wherein a bond between C and C represents a disulfide bond.) or a compound consisting of an amino acid sequence represented by Formula (2): 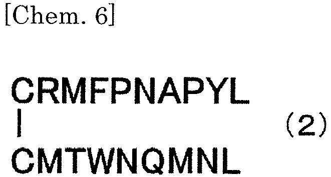
(wherein a bond between C and C represents a disulfide bond.) or a pharmaceutically acceptable salt thereof; and
a peptide consisting of an amino acid sequence selected from the group consisting of WAPVLDFAPPGASAYGSL (SEQ ID NO: 1), CWAPVLDFAPPGASAYGSL (SEQ ID NO: 2), and WAPVLDFAPPGASAYGSLC (SEQ ID NO: 3), or
a pharmaceutically acceptable salt thereof.

As a result, as a method for preparing a peptide emulsion formulation containing the peptide, the present inventors have found that the method may include a step of mixing an aqueous solution containing the compound and the like and the peptide and the like with an oily formulation and then applying vibration mixing, and a membrane emulsification step of passing the premixed solution after the vibration mixing through a membrane filter to emulsify the premixed solution. Hereinafter, the peptide emulsion formulation containing the above peptide may be referred to as a "desired peptide emulsion formulation" or simply as a "peptide emulsion formulation".

First, the peptide and the like used in the method for preparing a peptide emulsion formulation of the present invention will be described.

In the method for preparing a peptide emulsion formulation of the present invention,
a compound consisting of an amino acid sequence represented by Formula (1): 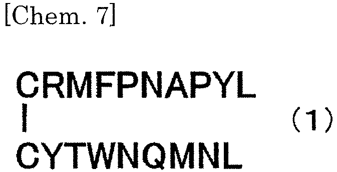 (wherein a bond between C and C represents a disulfide bond.) or
a compound consisting of an amino acid sequence represented by Formula (2): 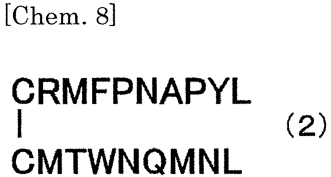
(wherein a bond between C and C represents a disulfide bond.) or a pharmaceutically acceptable salt thereof, and
a peptide consisting of an amino acid sequence selected from the group consisting of WAPVLDFAPPGASAYGSL (SEQ ID NO: 1), CWAPVLDFAPPGASAYGSL (SEQ ID NO: 2), and WAPVLDFAPPGASAYGSLC (SEQ ID NO: 3), and a peptide consisting of an amino acid sequence disclosed in WO 2018/181648 A or WO 2019/131722 A, or pharmaceutically acceptable salts thereof are used.

In the present specification, the left side of the peptide is an N-terminal, and each amino acid symbol indicates the following amino acid residues.
A: Alanine residue
R: Arginine residue
N: Asparagine residue
D: Aspartic acid residue
C: Cysteine residue
Q: Glutamine residue
E: Glutamic acid residue
G: Glycine residue
H: Histidine residue
I: Isoleucine residue
L: Leucine residue
K: Lysine residue
M: Methionine residue
F: Phenylalanine residue
P: Proline residue
S: Serine residue
T: Threonine residue
W: Tryptophan residue
Y: Tyrosine residue
V: Valine residue

The compound consisting of an amino acid sequence represented by the above Formula (1) is obtained by disulfide bonding between N-terminal cysteine residues of a peptide consisting of an amino acid sequence represented by CRMFPNAPYL (SEQ ID NO: 5) in which a cysteine residue is bonded to the N-terminal of a WT1 cancer antigen peptide represented by RMFPNAPYL (SEQ ID NO: 4) and a peptide represented by CYTWNQMNL (SEQ ID NO: 7) in which the second methionine residue from the N-terminal of the WT1 cancer antigen peptide represented by CMTWNQMNL (SEQ ID NO: 6) is modified to a tyrosine residue. The preparation method of the present invention can be used in a known method disclosed in, for example, WO 2014/157692 A or WO 2016/186177 A, or in a method and use described in International Patent Application No. PCT/JP 2019/39383.

The salt of the compound having an amino acid sequence represented by the above Formula (1), the salt of the compound having an amino acid sequence represented by the above formula (2), or the salt of the peptide having an amino acid sequence represented by WAPVLDFAPPGASAYGSL (SEQ ID NO: 1), CWAPVLDFAPPGASAYGSL (SEQ ID NO: 2), or WAPVLDFAPPGASAYGSLC (SEQ ID NO: 3) is not particularly limited as long as it is a pharmaceutically acceptable salt. Examples of the "salt" in the present invention include an acid addition salt and a base addition salt. Examples of the acid addition salt include inorganic acid salts such as hydrochloride, hydrobromide, sulfate, hydroiodide, nitrate, and phosphate, and organic acid salts such as citrate, oxalate, acetate, formate, propionate, benzoate, trifluoroacetate, maleate, tartrate, methanesulfonate, benzenesulfonate, and paratoluenesulfonate. Examples of the base addition salt include inorganic base salts such as sodium salt, potassium salt, calcium salt, magnesium salt, and ammonium salt, and organic base salts such as triethylammonium salt, triethanolammonium salt, pyridinium salt, and diisopropylammonium salt. Amino acid salts such as basic or acidic amino acids such as arginine, aspartic acid, and glutamic acid are further included.

The compound or the peptide of the present invention also includes a compound consisting of an amino acid sequence represented by the above Formula (1) or a salt thereof, a compound consisting of an amino acid sequence represented by the above Formula (2) or a salt thereof, a peptide consisting of an amino acid sequence selected from the group consisting of WAPVLDFAPPGASAYGSL (SEQ ID NO: 1), CWAPVLDFAPPGASAYGSL (SEQ ID NO: 2), and WAPVLDFAPPGASAYGSLC (SEQ ID NO: 3) or a hydrate of a salt thereof, or a solvate such as an ethanol solvate. Furthermore, the compound or peptide of the present invention includes any stereoisomer that can exist, such as any diastereomer or enantiomer, of a compound consisting of an amino acid sequence represented by the above Formula (1), a compound consisting of an amino acid sequence represented by the above Formula (2), or a salt thereof, or a peptide consisting of an amino acid sequence selected from the group consisting of WAPVLDFAPPGASAYGSL (SEQ ID NO: 1), CWAPVLDFAPPGASAYGSL (SEQ ID NO: 2), and WAPVLDFAPPGASAYGSLC (SEQ ID NO: 3), and a crystal form of any embodiment.

A compound consisting of an amino acid sequence represented by the above Formula (1) or the above Formula (2) or a salt thereof, and a peptide consisting of an amino acid sequence selected from the group consisting of WAPVLDFAPPGASAYGSL (SEQ ID NO: 1), CWAPVLDFAPPGASAYGSL (SEQ ID NO: 2), and WAPVLDFAPPGASAYGSLC (SEQ ID NO: 3) or a salt thereof can be produced, for example, by a known method disclosed in WO 2014/157692 A.

As described above, the present inventors have found the preparation method of the present invention in view of the fact that it is difficult to obtain a peptide emulsion formulation that is more stable for a long time by a known method or it is difficult to prepare the peptide emulsion formulation in a short time, particularly when the two types of peptides are used. Hereinafter, the preparation method of the present invention will be described.

### [Step of mixing aqueous solution containing the peptide with oily formulation, and then applying vibration mixing]

First, an aqueous solution containing the two peptides (peptide-containing aqueous solution) and an oily formulation are mixed with each other, and then vibration mixing is applied the mixture.

A ratio of the peptide in the peptide-containing aqueous solution can be, for example, 0.1 to 100 mg in 1 mL. The peptide-containing aqueous solution may further contain other pharmaceutically acceptable optional components in addition to the peptide as necessary. Examples of the optional component include a surfactant, a pH adjusting agent, a buffering agent, a solubilizer, a preservative, an excipient, a thickener, a stabilizer, a thickening agent, an antioxidant, an emulsifier, a dispersant, an isotonizing agent, and a chelating agent. One or more of these optional components may be contained as necessary. The content of the optional component may be appropriately set according to a purpose or the like. Examples of the method for preparing the peptide-containing aqueous solution include a method in which the peptide and the optional component are mixed, a method in which the solution obtained by the above method is lyophilized or spray-dried and dissolved in water again, and a method in which the lyophilized or spray-dried dry formulation is mixed with a solvent.

The "excipient" in the present invention generally refers to an excipient used in pharmaceutical formulations. Although not particularly limited, examples thereof include purified sucrose, glycine, lactose, glucose, maltose, sodium chloride, sucrose, mannitol, trehalose, and trehalose hydrate, and one or more of these can be used.

The "pH adjusting agent" in the present invention generally refers to a pH adjusting agent used in pharmaceutical formulations. Examples of the pH adjusting agent include one or more selected from the group consisting of an acid and a salt thereof and a base and a salt thereof. Specific examples thereof include inorganic acids such as hydrochloric acid, sulfuric acid, nitric acid, phosphoric acid, disodium phosphate, dipotassium phosphate, sodium dihydrogen phosphate, potassium dihydrogen phosphate, and trisodium phosphate or salts thereof, organic acids such as acetic acid, citric acid, tartaric acid, succinic acid, lactic acid, maleic acid, sodium acetate hydrate, sodium acetate anhydride, sodium citrate hydrate, sodium dihydrogen citrate, and sodium tartrate or salts thereof, inorganic bases such as sodium hydroxide, potassium hydroxide, and aqueous ammonia, and organic bases such as trometamol, histidine, L-arginine, and meglumine, and one or more of these can be used. It is preferably one or more selected from the group consisting of acids such as citric acid, lactic acid, tartaric acid, succinic acid, hydrochloric acid, sulfuric acid, and nitric acid, bases such as sodium hydroxide, potassium hydroxide, trometamol, histidine, L-arginine, and meglumine, and more preferably one or more selected from the group consisting of acids such as tartaric acid, succinic acid, and hydrochloric acid, bases such as sodium hydroxide, trometamol, L-arginine, and meglumine.

The "antioxidant" in the present invention refers to an antioxidant generally used in the pharmaceutical formulations. Specific examples thereof include methionine, ascorbic acid, sodium edetate, and sodium pyrosulfite, and one or more of these can be used.

The "solubilizer" in the present invention refers to a solubilizer generally used in the pharmaceutical formulations. Examples thereof include natural cyclodextrins such as α-cyclodextrin, β-cyclodextrin, γ-cyclodextrin, hydroxypropyl β-cyclodextrin, and sulfobutyl ether β-cyclodextrin, cyclodextrin derivatives such as hydroxyethyl-β-cyclodextrin (HE-β-CD), hydroxypropyl-β-cyclodextrin (HP-β-CD), methyl-β-cyclodextrin (M-β-CD), and sulfobutyl ether-β-cyclodextrin (SBE-β-CD), amines such as ethylenediamine, triethanolamine, and diethanolamine, acids such as aspartic acid, glycine, phosphoric acid, tartaric acid, acetic acid, citric acid, succinic acid, sodium L-arginine deoxycholic acid, and ursodesoxycholic acid, urea, ethyl urea, meglumine, ethanol, propylene glycol, polyethylene glycol, sodium salicylate, and nicotinic acid amide, and one or more of these can be used. It is preferably one or more selected from the group consisting of β-cyclodextrin, hydroxypropyl-β-cyclodextrin (HP-β-CD), tartaric acid, and succinic acid.

The "oily formulation" of the present invention to be mixed with the peptide-containing aqueous solution refers to a formulation having a nonaqueous solvent as a main solvent. The oily formulation contains a pharmaceutically acceptable oil. Examples of the pharmaceutically acceptable oil include a mineral oils such as liquid paraffin, paraffin, gelled hydrocarbon, and petrolatum, vegetable oils such as olive oil, safflower oil, soybean oil, camellia oil, corn oil, rapeseed oil, sunflower oil, cottonseed oil, and peanut oil, animal oils such as lard, squalane, and fish oil, and one or more of these can be used. The oily formulation may further contain other pharmaceutically acceptable optional components in addition to the oil. Examples of the optional component include a surfactant, a pH adjusting agent, a buffering agent, a solubilizer, a preservative, a thickener, a stabilizer, a thickening agent, an antioxidant, a dispersant, an isotonizing agent, and a chelating agent. One or more of these optional components may be contained as necessary. As the pH adjusting agent, the antioxidant, and the solubilizer, those described above can be used. The content of the optional component may be appropriately set according to a purpose or the like.

Examples of the surfactant include a nonionic surfactant, an anionic surfactant, a cationic surfactant, and a zwitterionic surfactant. Examples of the nonionic surfactant include polyoxyethylene alkyl ether, sorbitan fatty acid ester, polyoxyethylene hydrogenated castor oil, polyoxyethylene castor oil, polysorbate, polyoxyethylene polyoxypropylene glycol, sucrose fatty acid ester, and glycerin fatty acid ester. Examples of the anionic surfactant include sodium lauryl lactate. Examples of the cationic surfactant include benzalkonium chloride and benzethonium chloride. Examples of the zwitterionic surfactant include lecithin. As the surfactant, one or more of these can be used.

The oily formulation preferably contains the mineral oil and a plant-derived surfactant.

Examples of the oily formulation include an oily adjuvant as an oil emulsion that wraps an aqueous antigen solution with mineral oil to form and emulsify micelles. Examples of the oily adjuvant include liquid paraffin, lanolin, complete Freund's adjuvant (CFA), or incomplete Freund's adjuvant (IFA). Examples of the incomplete Freund's adjuvant include Montanide (For example, Montanide ISA 51).

"Mixing" in the present invention refers to the act of physically bringing two or more different types of components into a suitable homogeneous state. For example, after adding an equal amount of the oily formulation to the peptide-containing aqueous solution, a homogeneous state in which the mixture is "mixed" by "vibration mixing" by hand shaking for 10 seconds or more is obtained.

A mass ratio of the peptide-containing aqueous solution and the oily formulation to be mixed can be, for example, in the range of 1 : 10 to 10 : 1. The ratio is preferably in the range of 1 : 5 to 5 : 1, more preferably in the range of 1 : 2 to 2 : 1, and still more preferably 1 : 1.

The method for mixing the peptide-containing aqueous solution and the oily formulation may be either adding the oily formulation to the peptide-containing aqueous solution or adding the peptide-containing aqueous solution to the oily formulation.

The "vibration mixing" in the present invention refers to a method for mixing by applying vibration to an object to be mixed. Examples of a method of the vibration mixing include mixing in which a shaking method is a turning method, and a vertical movement method. Specific examples thereof include hand shaking and mixing by applying external vibration. In the case of the hand shaking, a hand shaking time, a hand shaking method, and the number of hand shaking are not limited, and the hand shaking time is, for example, several seconds to several tens of seconds, specifically, 5 seconds or more, 10 seconds or more, 15 seconds or more, 20 seconds or more, 25 seconds or more, 30 seconds or more, 35 seconds or more, 40 seconds or more, 45 seconds or more, 50 seconds or more, 60 seconds or more, 90 seconds or more, 2 minutes or more, 3 minutes or more, 4 minutes or more, or 5 minutes or more. The shaking may be performed for 5 minutes or less, 4 minutes or less, 3 minutes or less, 2 minutes or less, 90 seconds or less, 60 seconds or less, 50 seconds or less, 40 seconds or less, 30 seconds or less, 20 seconds or less, 10 seconds or less, or 5 seconds or less. The hand shaking method is not specifically limited, and examples thereof include shaking in a vertical direction, a horizontal direction, and an oblique direction, and preferably shaking up and down in the vertical direction. Specifically, the number of hand shaking may be 50 times or less, 40 times or less, 30 times or less, 25 times or less, 20 times or less, 15 times or less, 10 times or less, 5 times or less, 3 times or less, or 2 times, or 3 times or more, 5 times or more, 10 times or more, 15 times or more, 20 times or more, 25 times or more, 30 times or more, 40 times or more, or 50 times or more. Note that one round trip is counted as one hand shaking. Examples of the mixing by applying external vibration include mixing for 5 to 50 seconds in a vortex mixer (the shaking method is a swinging method, and the rotation speed is about 100 to 3000 rpm), and for example, mixing for 5 seconds, 10 seconds, 15 seconds, 20 seconds, 25 seconds, 30 seconds, 35 seconds, 40 seconds, 45 seconds, and 50 seconds can be performed. The speed of hand shaking is not specifically limited, and examples thereof include shaking at a speed of once, twice, or three times per second. Incidentally, the stirrer and the homomixer form a vortex but hardly generate vibration, and thus are not included in the method of vibration mixing of the present invention.

### [Membrane emulsification step of passing premixed solution after vibration mixing through membrane filter to emulsify premixed solution]

Next, the premixed solution after vibration mixing is passed through a membrane filter and emulsified to obtain an emulsion. In the premixed solution after the vibration mixing, an oil phase or an aqueous phase is not visually separated in the premixed solution, and the premixed solution may be passed through a membrane filter in a uniform cloudy liquid state. The time from the vibration mixing to the passage through the membrane filter is not limited to a specific time. Passing through the membrane filter may be performed within, for example, 30 minutes, 20 minutes, or 10 minutes after vibration mixing. It is preferable to pass through the membrane filter within 5 minutes, 4 minutes, 3 minutes, 2 minutes, or 1 minute after the vibration mixing, and it is more preferable to pass through the membrane filter immediately after the vibration mixing.

In the present invention, the premixed solution after vibration mixing is passed through the membrane filter once or twice or more. The number of times of passage can be, for example, 20 times or less, further 10 times or less, further 5 times or less, 4 times or less, 3 times or less, or twice or less from the viewpoint of improving workability. In addition, for example, when a disposable preparation tool is assumed, the membrane filter can be passed only once in one direction.

When the premixed solution is allowed to pass through the membrane filter twice or more, the premixed solution after vibration mixing may be allowed to pass through the membrane filter twice, 3 times, 4 times, 5 times, 6 times, 7 times, 8 times, 9 times, or 10 times or more, for example, by alternately combining the same direction or the opposite direction with respect to the membrane filter.

The "membrane filter" in the present invention refers to a porous filter having a thickness of several µm to several mm. When a membrane filter made of the following material is adopted, the membrane filter can have a thickness of, for example, 1 µm to 5000 pm, and examples thereof include, and are not limited to, 17 pm, 127 µm, or 150 µm.

Examples of the membrane filter include those formed of components containing fluororesins such as polytetrafluoroethylene (PTFE), polyvinylidene fluoride (PVDF), a tetrafluoroethylene-hexafluoropropylene copolymer (FEP), a tetrafluoroethylene-perfluoroalkyl vinyl ether copolymer (PFA), an ethylene-tetrafluoroethylene copolymer (ETFE), polychlorotrifluoroethylene (PCTFE), ethylene-chlorotrifluoroethylene copolymer (ECTFE), and polyvinyl fluoride (PVF), synthetic resins such as polyesters such as polyamides (PA) containing various aliphatic polyamides including nylon and aromatic polyamides, polyethylene terephthalate (PET), polybutylene terephthalate (PBT), polyethylene naphthalate (PEN), and polytributylene terephthalate (PTT), polyether sulfone (PES), an acrylic copolymer, polyethylene, and polyolefin containing polypropylene such as hydrophilic polypropylene; fibers such as cellulose acetate and MCE (cellulose mixed ester); glass fiber (GF); or a complex thereof. Examples of the membrane filter of the present invention include a porous membrane containing these components as a main skeleton component, and the membrane filter may be formed of one kind of component or two or more kinds of components.

Among them, the membrane filter is preferably formed of a component containing one or more selected from the group consisting of polytetrafluoroethylene, a fluororesin containing polyvinylidene fluoride, polyamide containing nylon, polyester, polyethersulfone, polypropylene, cellulose acetate, cellulose mixed ester, and glass fiber. In the membrane filter, the component is more preferably a main skeleton component.

The membrane filter is more preferably formed of a component containing one or more selected from the group consisting of polytetrafluoroethylene, a fluororesin containing polyvinylidene fluoride, polyamide, polyester, polypropylene, cellulose acetate, and glass fiber. It is further preferable that the component is a main skeleton component.

In the membrane filter, for example, the component may be formed of one or more (2 kinds, 3 kinds, 4 kinds, or 5 kinds) components, and one layer or a plurality of layers (2 layers, 3 layers, 4 layers, 5 layers, 6 layers, 7 layers, 8 layers, 9 layers or 10 layers) in which layers of the same component or different components overlap each other may be formed.

Among the membrane filters, the membrane filter is more preferably formed of a component containing a fluororesin, and among the fluororesins, one or more of polytetrafluoroethylene and polyvinylidene fluoride are particularly preferable. More particularly, the membrane filter is preferably formed of a component containing polytetrafluoroethylene (PTFE), and most preferably, the membrane filter preferably contains hydrophobic polytetrafluoroethylene (PTFE) as a main skeleton component.

When the main skeleton component of the membrane filter is polyamide, the pore size of the membrane filter can be, for example, in the range of 1 to 10 µm, and is, for example, 5 µm. In the case of polyester, the pore size of the membrane filter can be, for example, in the range of 5 to 30 pm, and the pore size may be, for example, 17 µm. When the main skeleton component of the membrane filter is PTFE, the pore size of the membrane filter can be, for example, within a range of 0.1 to 50 µm. Particularly, when the main skeleton component of the membrane filter is hydrophobic PTFE, the pore size of the membrane filter can be, for example, within a range of 0.3 to 50 µm, and is preferably within a range of 0.4 to 30 pm, more preferably 1 pm, 3 pm, or 5 pm, and still more preferably 5 µm. In the case of hydrophilic PTFE, the pore size of the membrane filter is, for example, in the range of 0.1 to 10 µm, for example, 0.1 pm, 0.2 pm, or 0.5 µm, and more preferably 0.2 µm. In the case of polyvinylidene fluoride (PVDF), the pore size of the membrane filter can be, for example, in the range of 0.1 to 3 pm, and the pore size may be, for example, 0.22 µm or 0.45 µm. When the main skeleton component of the membrane filter is glass fiber, the pore size of the membrane filter can be, for example, within a range of 0.5 to 5 µm, and examples thereof include 0.7 pm, 1 pm, and 3.1 µm. When the main skeleton component of the membrane filter is polypropylene, the pore size of the membrane filter is, for example, in the range of 0.1 to 5 µm, for example, 0.22 µm or 0.45 µm.

For the purpose of filter modification such as improvement of hydrophobicity, hydrophilicity, durability, and the like, the membrane filter may be one in which one or more layers of a component different from the main skeleton component, for example, a polymer layer, an artificial stratum corneum such as chitin or chitosan, and the like are coated on the surface of the main skeleton component, or one in which the surface of the main skeleton component is modified with a desired functional group with a silane coupling agent or the like. In addition, it is also possible to use a membrane filter in which the aforementioned other pharmaceutically acceptable optional components are present in advance in the voids of the membrane filter.

In the present invention, it is sufficient to pass the premixed solution after vibration mixing through the membrane filter once or twice or more, but the membrane filter can also be used by overlapping a plurality of layers (2 layers, 3 layers, 4 layers, 5 layers, 6 layers, 7 layers, 8 layers, 9 layers, or 10 layers) of membrane filters made of the same component or different components, for example, for the purpose of increasing physical strength.

According to the method of the present invention, a desired W/O type peptide emulsion formulation can be prepared at an early stage by a step including the step of applying vibration mixing and the membrane emulsification step. That is, it is possible to prepare a desired W/O type peptide emulsion in a short time without performing further steps described later.

As an operation method of membrane emulsification, a method for performing membrane emulsification by pulling out a premixed solution in a container through a filter, specifically, for example, a method for performing membrane emulsification by so-called "pulling out" in which a piston (also referred to as "plunger rod" or "administration holder") attached to an empty injection container to which a filter is attached is pulled out and suctions up the entire amount of the premixed solution in an injection container such as a syringe, a vial, or an ampoule. Alternatively, a method for performing membrane emulsification by extruding a premixed solution in an injection container such as a syringe through a filter, specifically, for example, a method for performing membrane emulsification by so-called "extrusion" in which a piston (also referred to as a "plunger rod" or an "administration holder") attached to an injection container containing the entire amount of the premixed solution is pushed, and the entire amount of the premixed solution is extruded into an empty injection container such as a syringe connected to the injection container and attached with a filter, a container such as a vial or an ampule, or extruded into the empty injection container such as a syringe or the container such as a vial or an ampule through a filter attached to the injection container to perform membrane emulsification may be used.

In the above, the method for preparing a W/O type peptide emulsion formulation has been described as an example, but the preparation method of the present invention is not limited thereto, and can also be applied to the preparation of an O/W type emulsion formulation in which the continuous phase is water and the discontinuous phase is oil droplets.

In order to obtain the W/O type or O/W type peptide emulsion formulation that is stable for a longer time, a step of reciprocating the emulsion obtained in the membrane emulsification step once or more between two connected containers without passing through a filter can be further included. Examples of the "container" used for the reciprocation include an injection container such as a syringe, a vial, and an ampoule. The material of the container is not limited, and for example, plastic or glass can be used. As the container, an injection container, for example, a syringe is preferably used. The "step of reciprocating" in the present invention refers to an action of moving a liquid present in one of the connected containers to the other container using a piston (also referred to as "plunger rod" or "administration holder") or the like, and moving the liquid to the original injection container again.

As a specific example of the step of reciprocating, for example, a filter needle at a tip of the injection container containing the emulsion is removed, a connector (also referred to as a "connection unit") is attached instead, air is removed as necessary, and then an empty injection container is attached to the other end of the connector. The emulsion is reciprocated once or more between two connected injection containers. In the present invention, the number of reciprocations is counted with one reciprocation as one.

The two containers used for reciprocating the emulsion between the containers may be containers having the same shape, or may be containers having different shapes.

Examples of the shape of the container include a container having an inner diameter of 1 to 10 mm and a volume of 1 to 3 mL, and a container having an inner diameter of 10 mm and a volume of 3 mL can be used. Examples of the shape of the connector include a connector having an inner diameter of 0.5 to 2 mm and a flow path length of 15 mm or less, and a connector having an inner diameter of 1 mm and a flow path length of 15 mm can be used.

As one embodiment of reciprocating the emulsion between the containers, the reciprocation is performed only once, that is, only one reciprocation. According to this embodiment, a more stable peptide emulsion formulation can be prepared in a short time. As another embodiment, the reciprocation may be performed several times or more, that is, several reciprocations or more. According to this embodiment, a peptide emulsion formulation that is more stable for a long time can be prepared. The term "several times or more" in the case of performing several or more reciprocations includes, for example, twice, 3 times, 4 times, 5 times, 6 times, 7 times, 8 times, 9 times, 10 times, 15 times, 20 times, 25 times, or 30 times, and can be, for example, 30 times or less, 25 times or less, 20 times or less, 15 times or less, 10 times or less, 5 times or less, 4 times or less, 3 times or less, or twice or more, 3 times or more, 4 times or more, 5 times or more, 10 times or more, 15 times or more, 20 times or more, 30 times or more, or 25 times or more.

The speed at which the emulsion is reciprocated between the containers may be a speed generally performed by a medical worker, and is usually about once, twice, or three times per second, but is not limited thereto.

The peptide emulsion formulation produced according to the above preferred embodiment, that is, after the membrane emulsification step and further subjected to the step of reciprocating once or more between two containers connected to each other without passing through a filter is obtained easily in a short time and has an effect of being stable for a long time. The term "stable for a long time" in the present invention refers to a state in which an oil phase or an aqueous phase is not separated from a peptide emulsion formulation during a period from preparation of the peptide emulsion formulation to administration to a patient in a general medical practice. Specific times are not limited, and include, for example, for about 1 hour, about 2 hours, about 3 hours, about 4 hours, about 5 hours, about 8 hours, about 12 hours, or about 24 hours, a state in which an oil phase or an aqueous phase is not separated from the prepared peptide emulsion formulation, and the like.

The method for preparing a peptide emulsion formulation of the present invention may further include a step of dispersing the peptide emulsion formulation using ultrasonic waves, a step of removing bubbles generated during the preparation, and the like, as necessary, in addition to the steps described above.

The "kit" in the present invention includes the two types of peptides, water for dissolution, an oily formulation, and a membrane filter, preferably the membrane filter described above, and may further include injection tools (which may include a plunger rod or the like) such as a syringe, an injection needle, a liquid feeding tube, and/or a container such as a vial or an ampoule.

The "device for preparing an emulsion" in the present invention may include at least a membrane filter, preferably the membrane filter described above, and for example, may further include injection containers (which may contain a plunger rod or the like) such as a syringe and/or a container such as a vial or an ampule, or may be used in combination thereof.

As another embodiment of the present invention, instead of the step of applying vibration mixing, an aqueous solution containing two types of peptides and an oily formulation can be placed in separate containers and connected with a filter interposed therebetween. In that case, a desired W/O type or O/W type peptide emulsion formulation can also be prepared by a membrane emulsification step in which an aqueous solution containing two peptides and/or an oily formulation are passed through a membrane filter once or twice or more to emulsify.

### EXAMPLES

Hereinafter, the present invention will be described more specifically with reference to Examples. The present invention is not limited by the following examples, and can be implemented with appropriate modifications within the scope that can be consistent with the above-described and later-described gist, and any of them is included in the technical scope of the present invention.

### [1. Study of Preliminary Mixing method]

### [1.1 Preparation of Example Emulsion 1]

A lyophilized formulation of a peptide represented by an amino acid sequence of WAPVLDFAPPGASAYGSL (SEQ ID NO: 1) and a lyophilized formulation of a peptide represented by the following Formula (1) were dissolved in water for injection to obtain 1.0 mL of an aqueous peptide solution having a peptide concentration of 3.5 mass%.
Formula (1):

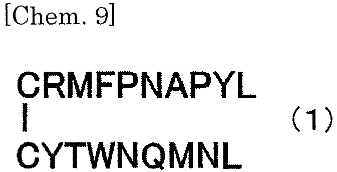

Then, 1.0 mL of an oily formulation (Montanide ISA51VG) was injected into a vial containing 1.0 mL of the aqueous peptide solution, and the vial was shaken by hand for about 10 seconds as premixing to obtain a premixed solution.

Thereafter, the entire amount of the premixed solution was subjected to membrane emulsification by pulling out a piston and suctioning up the solution in an empty syringe (available from B.BRAUN, volume 3.0 mL, inner diameter 10 mm) to which a filter needle (a membrane filter having a material and a pore size shown in Table 1) was attached to obtain an emulsion (emulsification operation method: pulling out).

As a membrane filter, polyamide (pore diameter: 5 µm, available from B. BRAUN, product number: FN-5019, filter needle), glass fiber (pore diameter: 3.1 pm, available from Thermo Scientific, product number: F2500 20, diameter: 30 mm), or PVDF (pore diameter: 0.45 pm, available from Millipore, product number: Hillex HV/SLHV033RS, diameter: 33 mm) was used. The passage through the membrane filter by sucking up with the syringe was only once.

### [1.2 Preparation of Comparative Example Emulsion 1]

In the preliminary mixing step, a stirrer (rotation speed: 620 rpm) was used instead of hand shaking, and then an emulsion was obtained by the pulling-out method. Comparative Example Emulsion 1 was obtained in the same manner as in the preparation of Example Emulsion 1 except for the premixing step.

### [1.3 Morphological Evaluation of Emulsion]

Only one drop of the resulting emulsion was slowly dropped into a 100 mL beaker containing about 50 mL of water. When the droplet was not dispersed in water and floated on the water surface in a droplet form, it was determined that a W/O emulsion was obtained. On the other hand, when the droplet was quickly dispersed in water, it was determined that an O/W emulsion was obtained. Evaluation was performed three times under the same conditions. Among the three times, when the W/O emulsion was obtained three times, it was evaluated as very good "⊙", when the W/O emulsion was obtained twice, it was evaluated as good "○", when the W/O emulsion was obtained once, it was evaluated as possible "△", and when the W/O emulsion was not obtained even once, it was evaluated as poor "×". The results are shown in Table 1.

**[Table 1]**

| Membrane materials of filter | Pore size of filter | Premixing method | Evaluation result |
|---|---|---|---|
| Polyamide | 5 µm | Hand shaking | ⊙ |
| Polyamide | 5 µm | Stirrer | × |
| Glass fiber | 3.1 µm | Hand shaking | ⊙ |
| Glass fiber | 3.1 µm | Stirrer | × |
| PVDF | 0.45 µm | Hand shaking | ⊙ |
| PVDF | 0.45 µm | Stirrer | × |

As shown in Table 1, it was possible to accurately prepare the W/O emulsion by performing vibration mixing as preliminary mixing.

### [2. Variations of Material and Pore Diameter of Filter]

### [2.2 Preparation of Example Emulsion 2]

A premixed solution was obtained by hand shaking in the same manner as in Example 1, and then the entire amount of the premixed solution was subjected to membrane emulsification by pulling out a piston and suctioning up the solution in a syringe (available from B.BRAUN, volume 3 mL, inner diameter 10 mm) to which a filter needle was attached, thereby obtaining an emulsion (emulsification operation method: pulling out). Alternatively, after the entire amount of the premixed solution was collected in a syringe, a filter was attached to the tip of the collected syringe, and the piston of the syringe containing the entire amount of the premixed solution was pushed to perform membrane emulsification, thereby obtaining an emulsion (emulsification operation method: extrusion).

Next, the used filter needle or filter attached to the syringe containing the emulsion was removed, and instead, a connector (inner diameter: 1 mm, flow path length: 15 mm) was connected to the syringe, and an empty syringe (available from B.BRAUN, volume 3 mL, inner diameter 10 mm) was connected to the other end of the connector. Then, the emulsion was reciprocated 20 times between the two syringes. Hereinafter, the reciprocating operation may be referred to as pumping. The same applies to the reciprocation in [4. Effect of pumping] described later.

As the filter needle or the filter, membrane filters having various materials and pore diameters shown in Table 2 were used. Nylon, cellulose acetate, glass fiber, and hydrophilic polypropylene available from Membrane Solutions were used, polyamide available from B. BRAUN was used, polyester available from FORTE GROW MEDICAL was used, and hydrophobic PTFE, PVDF, and cellulose mixed ester available from Millipore were used. Hydrophilic PTFE available from Advantec Toyo Kaisha, Ltd., polyethersulfone available from PALL, and an acrylic copolymer available from Becton Dickinson were used.

### [2.3 Morphological Evaluation of Emulsion]

Only one drop of the resulting emulsion was slowly dropped into a 100 mL beaker containing about 50 mL of water. When the droplet was not dispersed in water and floated on the water surface in a droplet form, it was determined that a W/O emulsion was obtained. On the other hand, when the droplet was quickly dispersed in water, it was determined that an O/W emulsion was obtained. Evaluation was performed three times for the same type of filter. Among the three times, when the W/O emulsion was obtained three times, it was evaluated as very good "⊙", when the W/O emulsion was obtained twice, it was evaluated as good "○", when the W/O emulsion was obtained once, it was evaluated as possible "△", and when the W/O emulsion was not obtained even once, it was evaluated as poor × The results are shown in Table 2.

**[Table 2]**

| Materials of membrane filter | Pore size of membrane filter | Emulsification operation method | Evaluation results |
|---|---|---|---|
| Nylon | 5 µm | Pulling out | ○ |
| Polyamide | 5 µm | Pulling out | ⊙ |
| | | Pushing | ○ |
| Polyester | 17 µm | Pulling out | ⊙ |
| | | Pushing | ⊙ |
| Hydrophobic polytetrafluoroethylene (PTFE) | 0.45 µm | Pushing | ⊙ |
| | 5 µm | Pulling out | ⊙ |
| | | Pushing | ⊙ |
| Hydrophilic polytetrafluoroethylene (PTFE) | 0.2 µm | Pushing | ⊙ |
| Polyvinylidene fluoride (PVDF) | 0.22 µm | Pulling out | ⊙ |
| | | Pushing | ⊙ |
| | 0.45 µm | Pulling out | ○ |
| | | Pushing | ⊙ |
| Cellulose mixed ester (MCE) | 0.45 µm | Pulling out | ○ |
| Cellulose acetate (CA) | 0.22 µm | Pulling out | ○ |
| | 0.45 µm | Pulling out | ⊙ |
| Glass fiber (GF) | 0.7 µm | Pushing | ○ |
| | 1 µm | Pulling out | ○ |
| | 3.1 µm | Pulling out | ⊙ |
| | | Pushing | ⊙ |
| Polypropylene (PP) | 0.22 µm | Pulling out | ⊙ |
| | 0.45 µm | Pulling out | ⊙ |
| | | Pushing | ○ |

As shown in the results of Table 2, preferably, by using a filter made of polyamide containing nylon, polyester, hydrophobic PEFE, hydrophilic PTFE, PVDF, a cellulose mixed ester, cellulose acetate, glass fiber, and hydrophilic polypropylene, it was possible to prepare a W/O emulsion by the method.

### [3. Evaluation of Reproducibility of Preparation Method of the Present Invention]

### [3.1 Preparation of Example Emulsion 3]

A premixed solution was obtained by hand shaking in the same manner as in Example 1, and then the entire amount of the premixed solution was subjected to membrane emulsification by pulling out a piston and suctioning up the solution in a syringe (available from B.BRAUN, volume 3 mL, inner diameter 10 mm) to which a membrane filter (Material: hydrophobic PTFE, pore size: 5 µm) was attached, thereby obtaining an emulsion (emulsification operation method: pulling out). As a filter of the hydrophobic PTFE, one available from Millipore was used.

### [3.2 Morphological Evaluation of Emulsion]

Only one drop of the resulting emulsion was slowly dropped into a 100 mL beaker containing about 50 mL of water. When the droplet was not dispersed in water and floated on the water surface in a droplet form, it was determined that a W/O emulsion was obtained. On the other hand, when the droplet was quickly dispersed in water, it was determined that an O/W emulsion was obtained. Then, a case where a W/O emulsion was possibly prepared was evaluated as good, and a case where a W/O emulsion was not possibly prepared was evaluated as poor.

### [3.3 Evaluation of Reproducibility of Preparation Method]

The preparation of the emulsion and the morphological evaluation of the emulsion were performed 10 times. As a result, it was possible to prepare a W/O emulsion in all 10 times. That is, the results of the evaluation of the morphology of all the emulsions were good 10 times, and it was found that a W/O emulsion was possibly prepared with good reproducibility by adopting the preparation method of the present invention.

### [4. Effect of Pumping]

### [4.1 Preparation of Example Emulsion 4]

A premixed solution was obtained by hand shaking in the same manner as in Example 1, and then the entire amount of the premixed solution was subjected to membrane emulsification by pulling out a piston and suctioning up the solution in a syringe (available from B.BRAUN, volume 3 mL, inner diameter 10 mm) to which a membrane filter shown in Table 3 was attached, thereby obtaining an emulsion (emulsification operation method: pulling out).

For some examples, the used filter needle attached to the syringe containing the obtained emulsion was removed, and instead, a connector (inner diameter: 1 mm, flow path length: 15 mm) was connected to the syringe, and another empty syringe (available from B.BRAUN, volume 3 mL, inner diameter 10 mm) was connected to the other end of the connector. Then, the emulsion was reciprocated once or more between the two syringes.

As the membrane filter shown in Table 3, polyamide available from B. BRAUN was used, polyester available from FORTE GROW MEDICAL was used, cellulose mixed ester available from Millipore, and hydrophobic PTFE available from Millipore were used.

### [4.2 Evaluation of Dispersion Stability of Emulsion]

The dispersion stability of the obtained emulsion was evaluated using a particle size distribution/dispersion stability analyzer (Device name: LUMiSizer (registered trademark), available from LUM). In the measurement, the emulsion was centrifuged at a rotation speed of 2000 rpm at 25°C for 2 hours or 3 hours, and a region up to about 25 mm from the bottom of the centrifugal separation cell was irradiated with measurement light every 5 minutes during the centrifugation, and the transmittance was evaluated from the profile of the transmittance at each position in the centrifugal direction of the region.

### (Measurement conditions)

Measurement cell: length 5 mm × width 10 mm × height 50 mm rectangular parallelepiped, optical path length 2 mm
Sample loading: 0.4mL
Wavelength of light source: 870nm

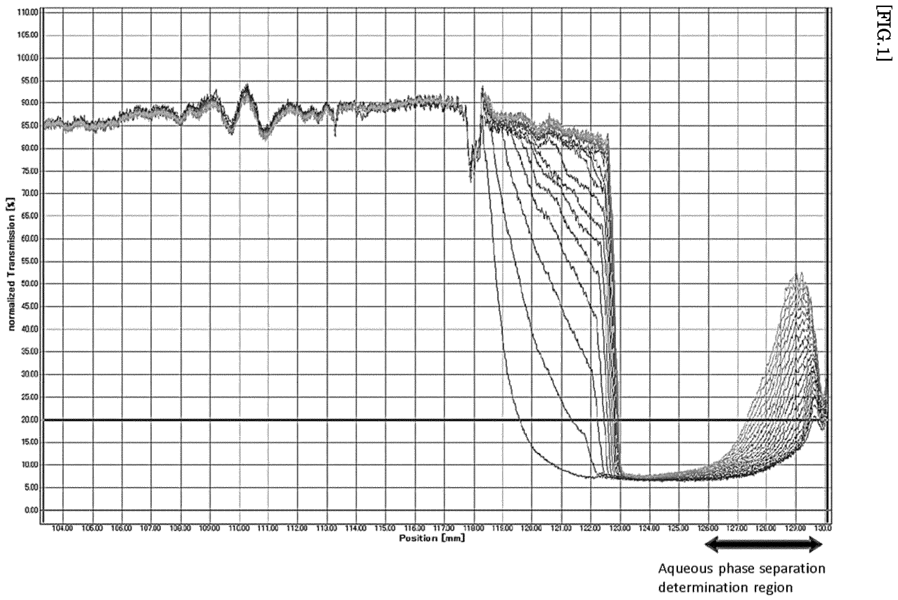

Among the results obtained in the above evaluation, the results in the case where hydrophobic PTFE is used and the number of pumping reciprocations is 0 are illustrated in Fig. 1 as an example. In Fig. 1, a vertical axis represents the transmittance, and a horizontal axis represents a position of a measurement cell as viewed from the side. Fig. 1 illustrates a superimposed transmittance profile obtained at regular time intervals from the start of centrifugation to after centrifugation for 2 hours. In this example, as the dispersion stability, it was evaluated that the aqueous phase was stable after centrifugation and was not separated. Specifically, as illustrated in Fig. 1, the degree of separation of the aqueous phase was determined by an aqueous phase separation determination region, that is, the amount of change in transmittance at a position of 123 to 130 mm on the horizontal axis (corresponding to 7 squares from the right end of the profile) in the profile, and the dispersion stability of the aqueous phase was evaluated. In this example, the transmittance after 2 hours of centrifugation was determined in the water phase separation determination region of the profile.

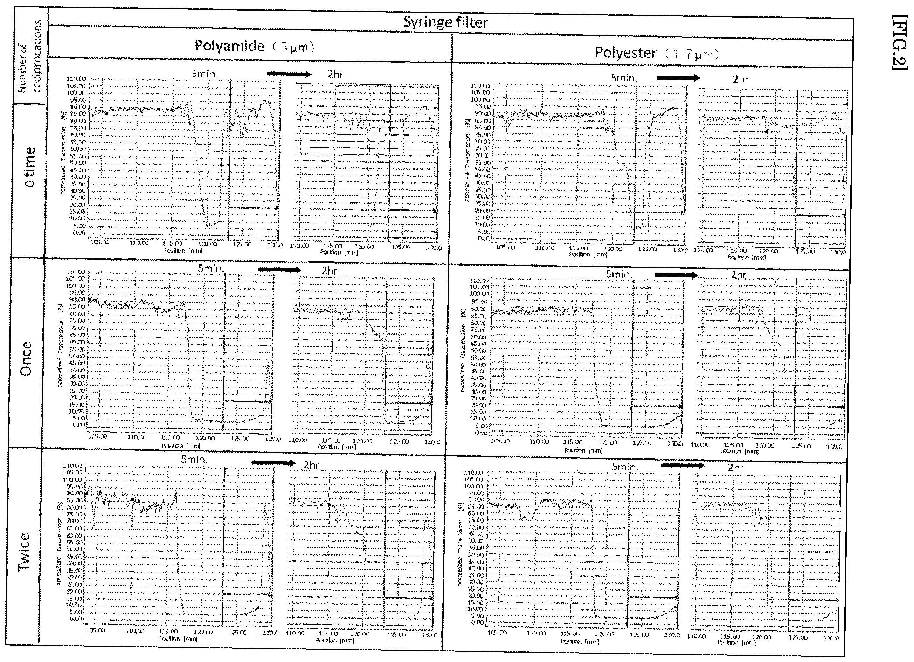

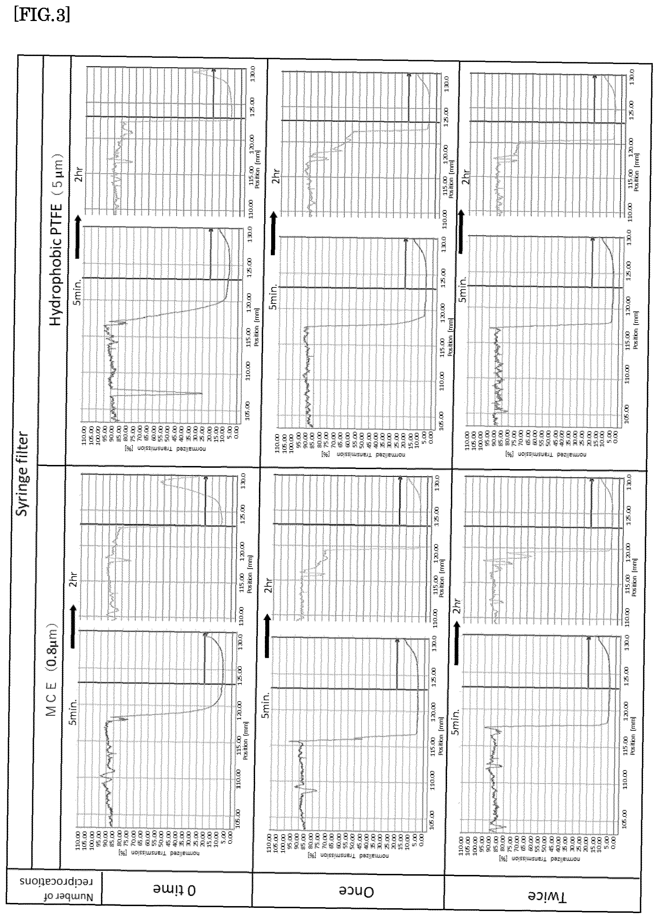

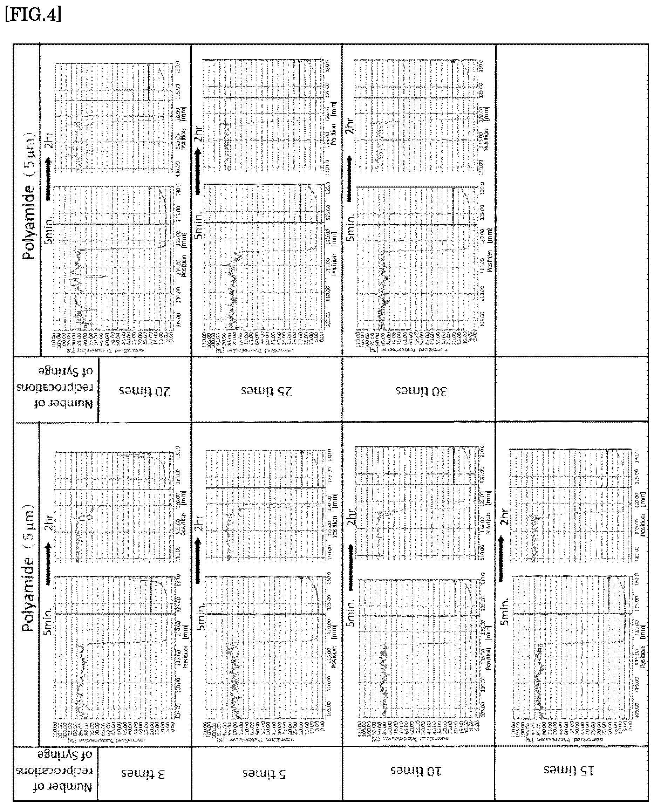

Figs. 2 and 3 show the profile after 5-minute centrifugation and the profile after 2-hour centrifugation in an example using each membrane filter. Among these profiles, the transmittance after 2-hour centrifugation in the water phase separation determination region was determined from the profile after 2-hour centrifugation. When the material of the filter was polyamide, the number of times of pumping was 0 to 3 times, 5 times, 10 times, 15 times, 20 times, 25 times, and 30 times to obtain a profile for each number of times. The profile is illustrated in Fig. 4. Table 3 shows the transmittance after 2-hour centrifugation in the cases of 0, once, 5 times, 10 times, and 15 times.

Table 3 shows the above transmittance. In the present Example, the case where the "transmittance after 2-hour centrifugation" was 15% or less was evaluated as being sufficiently suppressed in separation of the aqueous phase and being particularly excellent in long-term dispersion stability. This acceptance criterion corresponds to that the emulsion is stable for 8 hours or more after formulation in the medical field.

**[Table 3]**

| Number of pumping reciprocations | Transmittance (%) after 2-hour centrifugation for each filter material | | | |
|---|---|---|---|---|
| | Polyamide (pore size 5 µm) | Polyester (pore size 17 µm) | Cellulose mixed ester (pore size 0.8 µm) | Hydrophobic (PTFE) (pore size 5 µm) |
| 0 | 83.64 | 83.19 | 20.58 | 11.59 |
| 1 | 12.78 | 6.61 | 6.68 | 6.86 |
| 5 | 6.33 | - | - | - |
| 10 | 5.96 | - | - | - |
| 15 | 6.15 | - | - | - |

In the results of Fig. 2, Fig. 3 and Table 3, it was shown that separation of the aqueous phase is sufficiently suppressed by preferably performing pumping, and an emulsion formulation having more excellent dispersion stability over a long period of time is obtained. In addition, Fig. 4 illustrates that the dispersion stability is further improved by performing pumping one or more times as compared with the case without pumping.

## Claims

1. A method for preparing a peptide emulsion formulation comprising:
a step of mixing an aqueous solution containing a compound consisting of an amino acid sequence represented by Formula (1): 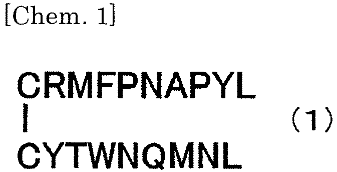
(wherein a bond between C and C represents a disulfide bond) or a pharmaceutically acceptable salt thereof, and a peptide consisting of an amino acid sequence represented by WAPVLDFAPPGASAYGSL (SEQ ID NO: 1) or a pharmaceutically acceptable salt thereof with an oily formulation and applying vibration mixing; and
a membrane emulsification step of passing a premixed solution after the vibration mixing through a membrane filter to emulsify the premixed solution.

2. The method for preparing a peptide emulsion formulation according to claim 1,
wherein in the membrane emulsification step, the premixed solution after the vibration mixing is caused to pass through the membrane filter once or twice or more to emulsify the premixed solution.

3. The method for preparing a peptide emulsion formulation according to claim 1,
wherein in the membrane emulsification step, the premixed solution after the vibration mixing is caused to pass through the membrane filter once to emulsify the premixed solution.

4. The method for preparing a peptide emulsion formulation according to any one of claims 1 to 3, further comprising:
a step of reciprocating an emulsion obtained in the membrane emulsification step once or several times or more between two containers connected without passing through a membrane filter.

5. The method for preparing a peptide emulsion formulation according to claim 4, wherein the number of reciprocations is one.

6. The method for preparing a peptide emulsion formulation according to any one of claims 1 to 5, wherein the vibration mixing is shaking by hand.

7. The method for preparing a peptide emulsion formulation according to any one of claims 1 to 6, wherein the membrane filter is formed of a component containing one or more selected from the group consisting of polyamide, polyester, fluororesin, polyethersulfone, polypropylene, cellulose acetate, cellulose mixed ester, and glass fiber.

8. The method for preparing a peptide emulsion formulation according to any one of claims 1 to 7, wherein the membrane filter is formed of a component containing one or more selected from the group consisting of polyamide, polyester, fluororesin, polypropylene, cellulose acetate, and glass fiber.

9. The method for preparing a peptide emulsion formulation according to claim 7 or 8, wherein the fluororesin is one or more selected from the group consisting of polytetrafluoroethylene and polyvinylidene fluoride.

10. The method for preparing a peptide emulsion formulation according to any one of claims 7 to 9, wherein the fluororesin is
polytetrafluoroethylene.

11. The method for preparing a peptide emulsion formulation according to any one of claims 1 to 10, wherein the oily formulation is incomplete Freund's adjuvant.

12. The method for preparing a peptide emulsion formulation according to any one of claims 1 to 11, wherein the oily formulation is Montanide.

13. The method for preparing a peptide emulsion formulation according to any one of claims 1 to 12, wherein the peptide emulsion formulation is a W/O type peptide emulsion formulation.

14. The method for preparing a peptide emulsion formulation according to any one of claims 1 to 13, wherein the peptide emulsion formulation contains an excipient.

15. The method for preparing a peptide emulsion formulation according to claim 14, wherein the excipient contains one or more selected from the group consisting of purified sucrose, glycine, lactose, glucose, maltose, sodium chloride, sucrose, mannitol, trehalose, and trehalose hydrate.

16. The method for preparing a peptide emulsion formulation according to any one of claims 1 to 15, wherein the peptide emulsion formulation contains a pH adjusting agent.

17. The method for preparing a peptide emulsion formulation according to claim 16, wherein the pH adjusting agent contains one or more selected from the group consisting of citric acid, lactic acid, tartaric acid, hydrochloric acid, sulfuric acid, nitric acid, succinic acid, sodium hydroxide, potassium hydroxide, tromethamol, histidine, L-arginine, and meglumine.

18. The method for preparing a peptide emulsion formulation according to claim 16 or 17, wherein the pH adjusting agent contains one or more selected from the group consisting of tartaric acid, succinic acid, hydrochloric acid, sodium hydroxide, trometamol, L-arginine, and meglumine.

19. The method for preparing a peptide emulsion formulation according to any one of claims 1 to 18, wherein the peptide emulsion formulation contains an antioxidant.

20. The method for preparing a peptide emulsion formulation according to claim 19, wherein the antioxidant contains one or more selected from the group consisting of methionine, ascorbic acid, sodium edetate, and sodium pyrosulfite.

21. The method for preparing a peptide emulsion formulation according to claim 19 or 20, wherein the antioxidant is methionine.

22. The method for preparing a peptide emulsion formulation according to any one of claims 1 to 21, wherein the peptide emulsion formulation contains a solubilizer.

23. The method for preparing a peptide emulsion formulation according to claim 22, wherein the solubilizer contains one or more selected from the group consisting of natural cyclodextrins such as α-cyclodextrin, β-cyclodextrin, γ-cyclodextrin, hydroxypropyl β-cyclodextrin, and sulfobutyl ether β-cyclodextrin, cyclodextrin derivatives such as hydroxyethyl-β-cyclodextrin (HE-β-CD), hydroxypropyl-β-cyclodextrin (HP-β-CD), methyl-β-cyclodextrin (M-β-CD), and sulfobutyl ether-β-cyclodextrin (SBE-β-CD), amines such as ethylenediamine, triethanolamine, and diethanolamine, acids such as aspartic acid, glycine, phosphoric acid, tartaric acid, acetic acid, citric acid, succinic acid, sodium L-arginine deoxycholic acid, and ursodesoxycholic acid, urea, ethyl urea, meglumine, ethanol, propylene glycol, polyethylene glycol, sodium salicylate, and nicotinic acid amide.

24. The method for preparing a peptide emulsion formulation according to claim 22 or 23, wherein the solubilizer contains one or more selected from the group consisting of β-cyclodextrin, hydroxypropyl-β-cyclodextrin (HP-β-CD), tartaric acid, and succinic acid.
